(19) Europäisches Patentamt
European Patent Office
Office européen des brevets

(11) Veröffentlichungsnummer: **0 318 781 B1**

(12) **EUROPÄISCHE PATENTSCHRIFT**

(45) Veröffentlichungstag der Patentschrift: **23.06.93**

(51) Int. Cl.5: **C07C 217/84**

(21) Anmeldenummer: **88119292.6**

(22) Anmeldetag: **21.11.88**

# (54) Am aromatischen Kern und an der Seitenkette Fluoratome enthaltende Alkoxyaniline und Verfahren zur Herstellung von am aromatischen Kern und an der Seitenkette Fluoratome enthaltenden Alkoxyanilinen.

(30) Priorität: **01.12.87 DE 3740636**

(43) Veröffentlichungstag der Anmeldung:
**07.06.89 Patentblatt 89/23**

(45) Bekanntmachung des Hinweises auf die Patenterteilung:
**23.06.93 Patentblatt 93/25**

(84) Benannte Vertragsstaaten:
**CH DE FR GB IT LI**

(56) Entgegenhaltungen:
**EP-A- 0 235 089**
**EP-A- 0 277 748**
**EP-A- 0 318 782**

(73) Patentinhaber: **BAYER AG**

**W-5090 Leverkusen 1 Bayerwerk(DE)**

(72) Erfinder: **Marhold, Albrecht, Dr.**
**Carl-Duisberg-Strasse 329**
**W-5090 Leverkusen 1(DE)**

EP 0 318 781 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der am Prioritätstag der vorliegenden Patentanmeldung noch nicht veröffentlichten EP-A 277 748 ist die Verbindung 2-Fluor-4-tetrafluorethoxy-anilin beschrieben und Verfahren zu deren Herstellung bei denen man entweder 2-Fluor-4-hydroxy-nitrobenzol zunächst mit Tetrafluorethylen umsetzt und danach reduziert oder 2-Fluor-4-hydroxy-nitrobenzol zunächst reduziert und dann mit Tetrafluorethylen umsetzt oder o-Fluornitrobenzol zu 3-Amino-4-fluor-phenol reduziert und dieses mit Tetrafluorethylen umsetzt.

Die vorliegende Erfindung betrifft neue, am aromatischen Kern und an der Seitenkette Fluoratome enthaltende Alkoxyaniline der Formel

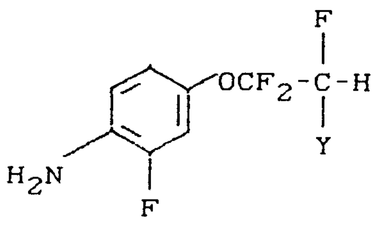

(I),

in der

Y für Fluor, Chlor oder Trifluormethyl steht,

wobei die Verbindung 2-Fluor-4-tetrafluorethoxy-anilin ausgenommen ist.

Verbindungen der Formel (I) sind :

2-Fluor-4-trifluorchlorethoxy-anilin und 2-Fluor-4-hexafluorpropoxy-anilin.

Ein allgemein anwendbares Verfahren zur Herstellung von Verbindungen der Formel (I)

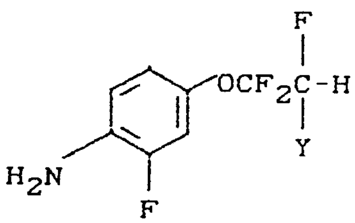

(I)

in der

Y für Fluor, Chlor oder Trifluormethyl steht,

mit Ausnahme der Verbindung 2-Fluor-4-tetrafluorethoxyanilin,

ist dadurch gekennzeichnet, daß man ein Fluorphenol der Formel (II)

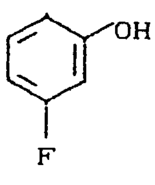

(II),

mit einer Trifluorethylen-Verbindung der Formel (III)

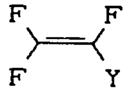

(III),

in der

Y die bei Formel (I) angegebene Bedeutung hat,

umsetzt und die dabei erhältliche Verbindung der Formel (IV)

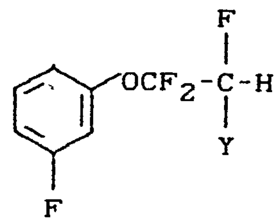

(IV),

in der

Y die bei Formel (I) angegebene Bedeutung hat,

nitriert und anschließend reduziert.

Die in dieses erfindungsgemäße Verfahren einzusetzenden Verbindungen der Formeln (II) und (III) sind bekannt (siehe z.B. Houben-Weyl, Band 5E (1985), Seite 14) oder können analog dazu hergestellt werden.

Die Umsetzung einer Verbindung der Formel (II) mit einer Verbindung der Formel (III) kann beispielsweise so erfolgen, daß man die Verbindung der Formel (II) in einem Lösungsmittel (z.B. Aceton, Dioxan oder Dimethylformamid) in Gegenwart eines Alkalihydroxids vorlegt und bei Temperaturen von 40 bis 80 °C die Verbindung der Formel (III) einleitet. Die Umsetzung ist im allgemeinen dann beendet, wenn keine Verbindung der Formel (III) mehr aufgenommen wird Das gebildete Produkt der Formel (IV) kann beispielsweise durch Destillation oder Extraktion aus dem Reaktionsgemisch isoliert werden.

Die daran anschließende Nitrierung kann mit üblichen Nitriermitteln durchgeführt werden, beispielsweise mit Gemischen aus Salpeter- und Schwefelsäure. Die Temperatur kann dabei beispielsweise im Bereich von 0 bis 80 °C liegen, vorzugsweise liegt sie bei 20 bis 50 °C. Das Nitriermittel kann man beispielsweise in Mengen einsetzen, bei denen im Reaktionsgemisch 0,8 bis 1,5 Mole Nitrieragens pro Mol der zu nitrierenden Verbindung gebildet werden. Vorzugsweise läßt man sich 1 bis 1,1 Mol Nitrieragens pro Mol der zu nitrierenden Verbindung bilden. Die Nitrierung kann gegebenenfalls in Gegenwart eines inerten organischen Lösungsmittels durchgeführt werden. Geeignet sind beispielsweise Methylenchlorid, Pentan und Hexan. In manchen Fällen kann mit bestimmten Lösungsmitteln die Isomerenverteilung im Reaktionsprodukt beeinflußt werden.

Die daran anschließende Reduktion kann chemisch, d.h. beispielsweise mit reduzierend wirkenden Metallen oder Metallsalzen durchgeführt werden. Geeignet sind beispielsweise Eisen, Zink, Zinn, Zinn(II)-chlorid und Titan(III)-chlorid und Sulfide. Derartige Reduktionsmittel werden vorzugsweise in der stöchiometrisch erforderlichen Menge eingesetzt. Für eine derartige Reduktion können die Nitroverbindungen z.B. so eingesetzt werden, wie sie bei der Nitrierung anfallen oder danach isoliert werden. Die Reduktion kann man auch katalytisch mit Wasserstoff durchführen, wobei z.B. Katalysatoren eingesetzt werden können, die Metalle enthalten oder daraus bestehen. Geeignet sind beispielsweise die Metalle der 8. Nebengruppe des periodischen Systems der Elemente, insbesondere Palladium, Platin und Nickel. Die Metalle können in elementarer Form oder in Form von Verbindungen vorliegen, sowie in besonders aktivierten Formen, z.B. in Form von Raney-Metallen oder aufgebracht als Metall oder Metallverbindung auf Trägermaterialien. Bevorzugt ist Raney-Nickel, Palladium auf Kohle und Palladium auf Aluminiumoxid.

Vorzugsweise wird die katalytische Reduktion in Gegenwart eines Lösungsmittels durchgeführt. Geeignet sind beispielsweise Alkohole und Ether, wie Methanol, Ethanol und Tetrahydrofuran sowie Kohlenwasserstoffe. Die katalytische Reduktion kann beispielsweise bei Temperaturen im Bereich 0 bis 80 °C und beispielsweise bei Wasserstoffdrucken im Bereich 1 bis 100 bar durchgeführt werden. Überschüsse an Wasserstoff sind im allgemeinen nicht kritisch.

Für die katalytische Reduktion werden vorzugsweise säurefreie Nitroverbindungen eingesetzt. Gegebenenfalls sind letztere also von Säuren zu befreien, z.B. durch Waschen mit Wasser oder Neutralisation mit einer Base.

Die Aufarbeitung des nach der chemischen Reduktion oder der katalytischen Hydrierung vorliegenden Reaktionsgemisches kann beispielsweise so erfolgen, daß man zunächst gegebenenfalls vorhandene feste Bestandteile abfiltriert und das Filtrat, gegebenenfalls nach einer Wäsche mit Wasser, destilliert.

Falls als Reaktionsprodukt ein Gemisch von Isomeren anfällt, kann man dieses im allgemeinen durch Feindestillation trennen.

Eine Variante dieses Verfahrens zur Herstellung von Verbindungen der Formel (I) ist dadurch gekennzeichnet, daß man eine Verbindung der Formel (V)

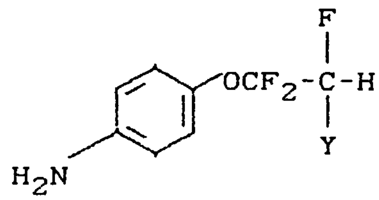

(V),

in der

Y die bei Formel (I) angegebene Bedeutung hat

mit Natriumnitrit in Gegenwart einer Fluor enthaltenden Protonensäure umsetzt, so eine Verbindung der Formel (IV) erhält und diese nitriert und anschließend reduziert.

Verbindungen der Formel (V) sind bekannt (siehe z.B. Houben-Weyl, Band 5E (1985), Seite 16) oder können analog dazu hergestellt werden.

Die Umsetzung mit Natriumnitrit in Gegenwart einer Fluor enthaltenden Protonensäure kann beispielsweise mit 0,9 bis 1,2 Molen Natriumnitrit pro Mol der Verbindung der Formel (V) und beispielsweise in Gegenwart von Fluorwasserstoff oder Tetrafluorborsäure erfolgen. Die Fluor enthaltende Protonensäure kann beispielsweise in Mengen von 1 bis 20 Mol eingesetzt werden. Geeignete Temperaturen für diese Umsetzung sind beispielsweise solche im Bereich von -10 bis +5 °C für die Diazotierung und 20 bis 180 °C für die Zersetzung des Diazoniumsalzes.

Die Verbindung der Formel (IV) kann aus dem Reaktionsgemisch beispielsweise durch Destillation oder Wasserdampfdestillation isoliert und gereinigt werden.

Die Nitrierung und Reduktion kann erfolgen wie es weiter oben detailliert beschrieben worden ist.

Bei den neuen, am aromatischen Kern und an der Seitenkette Fluoratome enthaltenden Alkoxyanilinen der Formel (I) handelt es sich um wichtige Zwischenprodukte. Beispielsweise kann man diese mit Benzoyliso(thio)cyanaten umsetzen und so substituierte Benzoyl(thio)harnstoffe der Formel (VIII) erhalten

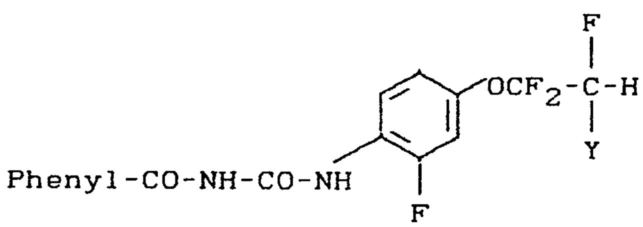

(VIII),

in der

Y die bei Formel (I) angegebene Bedeutung hat und

Phenyl für einen unsubstituierten oder substituierten Phenylrest steht. Falls dieser Phenylrest substituiert ist kann er beispielsweise unabhängig voneinander mit 1 bis 5 Fluoratomen, 1 bis 4 Chloratomen, 1 bis 2 Bromatomen, 1 bis 2 Iodatomen und/oder einer Nitro-, einer Methyl- und/oder einer Trifluormethylgruppe substituiert sein. Die Verbindungen der Formel (VIII) haben Eigenschaften, die eine Verwendung als Schädlingsbekämpfungsmittel ermöglichen. Sie zeigen insbesondere gute arthropodizide Eigenschaften und eine sehr starke insektizide Wirksamkeit.

Man kann die erfindungsgemäßen Verbindungen der Formel (I) z.B. auch mit Acrylnitril unter Diazotierungsbedingungen umsetzen, so α-Chlor-β-phenyl-propionitrile erhalten, deren Phenylanteil (mit Ausnahme der $NH_2$-Gruppe) in gleicher Weise substituiert ist wie im jeweils eingesetzten erfindungsgemäßen Produkt, diese durch Dehydrohalogenierung in das entsprechende Zimtsäurenitril überführen und daraus durch Umsetzung mit Sulfonylmethylisocyaniden 3-Cyano-4-phenyl-pyrrole gewinnen, deren Phenylteil (mit Ausnahme der $NH_2$-Gruppe) in gleicher Weise substituiert ist wie in der jeweils eingesetzten erfindungsgemäßen Verbindung der Formel (I). Derartige 3-Cyano-4-phenyl-pyrrole weisen eine besonders gute Wirksamkeit als Fungizide auf, insbesondere zum Schutz von Pflanzen gegen Plasmodiophoromycetes, Oomycetes, Chrytridiomycetes, Zygomycetes, Ascomycetes, Basidiomycetes und Deuteromycetes.

Verbindungen der Formel (VIII) und die zuvor beschriebenen 3-Cyano-4-phenyl-pyrrole, deren Herstellung und deren Verwendung als Pflanzenschutzmittel sind Gegenstand von separaten eigenen Patentanmeldungen.

Beispiele

Beispiel 1

a) 100 g 3-Fluorphenol wurden in 300 ml Aceton zusammen mit 20 g Natriumhydroxid bei 50 bis 55 °C vorgelegt und die Lösung mit Hexafluorpropen gesättigt (bis zum Ende der Gasabsorption). Anschließend wurde das Aceton zum größten Teil abdestilliert und der Rückstand mit 300 ml Wasser versetzt. Die organische Phase wurde abgetrennt und destilliert. Bei einem Siedepunkt von 56 bis 58 °C bei 20 mbar fielen 115 g 3-Fluorphenyl-hexafluorpropylether mit einem Brechungsindex $n_D^{20}$ von 1,3850 an.
b) In einer Rührapparatur wurden 305 g des gemäß a) hergestellten Produkts vorgelegt und 400 ml trockenes Dichlormethan zugegeben. Dann tropfte man 110 g Nitriersäure (33 % Salpetersäure, 67 % Schwefelsäure) bei 20 °C zu, rührte 1 Stunde bei dieser Temperatur und anschließend für eine weitere Stunde bei 40 °C nach. Die abgekühlte Reaktionsmischung wurde auf Eis gegossen und dann, nach gründlichem Durchmischen, die organische Phase abgetrennt. Die fraktionierte Destillation der organischen Phase lieferte bei einem Siedepunkt von 92 bis 95 °C bei 10 mbar 135 g (2-Nitro-5-fluor-phenyl)-hexafluorpropylether mit einem Brechungsindex $n_D^{20}$ von 1,4270, einen Zwischenlauf von 43 g und bei einem Siedepunkt von 104 bis 105 °C bei 10 mbar 87 g (4-Nitro-3-fluor-phenyl)-hexafluorpropylether mit einem Brechungsindex $n_D^{20}$ von 1,4325.
c) In einer Hydrierapparatur wurden die 87 g (4-Nitro-3-fluor-phenyl)-hexafluorpropylether, die gemäß b) erhalten wurden, in 380 ml Tetrahydrofuran zusammen mit 10 g Raney-Nickel vorgelegt und bei 25 bis 45 °C mit 30 bar Wasserstoff hydriert, bis kein Wasserstoff mehr verbraucht wurde. Nach dem Entspannen des Autoklaven wurde das Reaktionsgemisch filtriert und das Filtrat destilliert. Im Siedebereich von 105 bis 107 °C bei 20 mbar gingen 59 g 2-Fluor-4-hexafluorpropoxy-anilin mit einem Brechungsindex $n_D^{20}$ von 1,4230 über.

Beispiel 2

a) Es wurde verfahren wie in Beispiel 1a), jedoch wurde anstelle von Hexafluorpropen 150 g Trifluorchlorethylen eingesetzt. So wurden 139 g 3-Fluorphenyl-trifluorchlorethyl-ether mit einem Siedepunkt von 70 bis 73 °C bei 22 mbar und einem Brechungsindex $n_D^{20}$ von 1,4325 erhalten.
b) Es wurde verfahren wie in Beispiel 1 b), jedoch wurden 118 g des gemäß a) erhaltenen 3-Fluorphenyltrifluorchlorethylethers und 95 g Nitriersäure eingesetzt und 125 g einer Mischung bestehend aus 58,4 Gew.-% (2-Nitro-5-fluorphenyl)-trifluorchlorethyl-ether und 41,6 Gew.-% (4-Nitro-3-fluorphenyl)-trifluorchlorethyl-ether im Siedebereich 126 bis 138 °C bei 22 mbar isoliert.
c) Es wurde verfahren wie in Beispiel 1 c), jedoch wurden 125 g der gemäß b) erhaltenen Mischung eingesetzt. Mit einem Siedepunkt von 93 bis 95 °C bei 10 mbar gingen 38 g 4-Fluor-2-trifluorchlorethoxy-anilin ($n_D^{20}$ : 1,4720) und mit einem Siedepunkt von 99 bis 101 °C bei 10 mbar 29 g 2-Fluor-4-trifluorchlorethoxy-anilin über.

## Patentansprüche

**1.** Am aromatischen Kern und in der Seitenkette Fluoratome enthaltende Alkoxyaniline der Formel

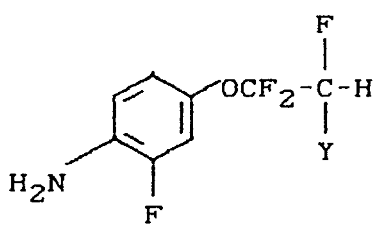

(I),

in der
Y für Fluor, Chlor oder Trifluormethyl steht,
wobei die Verbindung 2-Fluor-4-tetrafluorethoxyanilin ausgenommen ist.

2. Verfahren zur Herstellung von Verbindungen der Formel (I)

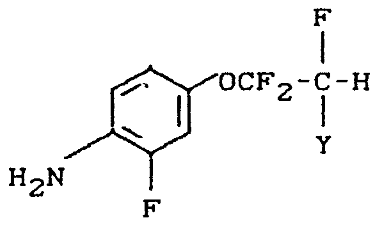

(I),

in der

Y für Fluor, Chlor oder Trifluormethyl steht,

mit Ausnahme der Verbindung 2-Fluor-4-tetrafluorethoxyanilin, dadurch gekennzeichnet, daß man ein Fluorphenol der Formel (II)

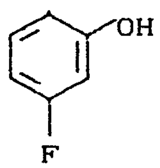

(II),

mit einer Trifluorethylen-Verbindung der Formel (III)

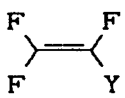

(III),

in der

Y die bei Formel (I) angegebene Bedeutung hat,

umsetzt und die dabei erhältliche Verbindung der Formel (IV)

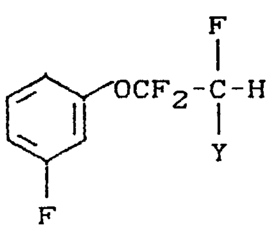

(IV),

in der

Y die bei Formel (I) angegebene Bedeutung hat,

nitriert und anschließend reduziert.

3. Verfahren zur Herstellung von Verbindungen der Formel (I), dadurch gekennzeichnet, daß man eine Verbindung der Formel (V)

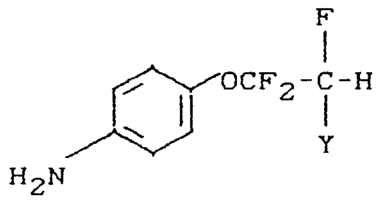

(V),

in der

Y die bei Formel (I) angegebene Bedeutung hat,

mit Natriumnitrit in Gegenwart einer Fluor enthaltenden Protonensäure umsetzt, so eine Verbindung der Formel (IV) erhält und diese nitriert und anschließend reduziert.

**Claims**

1. Alkoxyanilines containing fluorine atoms in the aromatic nucleus and in the side chain, of the formula

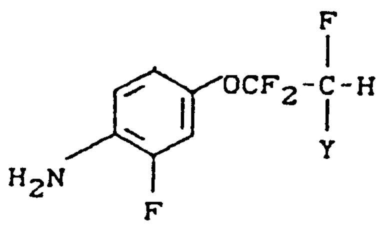

(I),

in which

Y represents fluorine, chlorine or trifluoromethyl,

with the exception of the compound 2-fluoro-4-tetrafluoroethoxyaniline.

2. Process for the preparation of compounds of the formula (I)

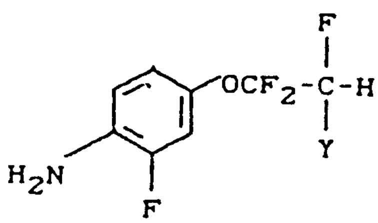

(I),

in which

Y represents fluorine, chlorine or trifluoromethyl,

with the exception of the compound 2-fluoro-4-tetrafluoroethoxyaniline, characterised in that a fluorophenol of the formula (II)

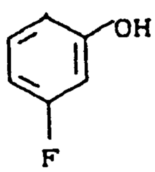

(II),

is reacted with a trifluoroethylene compound of the formula (III)

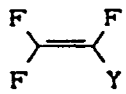

(III),

in which

Y has the meaning indicated in formula (I)

and the compound thus obtainable of the formula (IV)

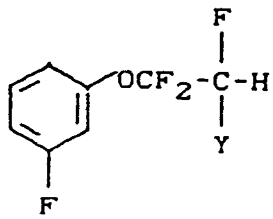

(IV),

in which

Y has the meaning indicated in formula (I)

is nitrated and subsequently reduced.

**3.** Process for the preparation of compounds of the formula (I), characterised in that a compound of the formula (V)

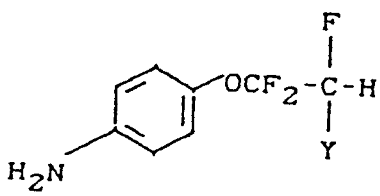

(V),

in which

Y has the meaning indicated in formula (I)

is reacted with sodium nitrite in the presence of a fluorine-containing protonic acid, a compound of the formula (IV) is thus obtained and this is nitrated and subsequently reduced.

**Revendications**

**1.** Alkoxyanilines contenant des atomes de fluor sur le noyau aromatique et dans la chaîne latérale, de formule

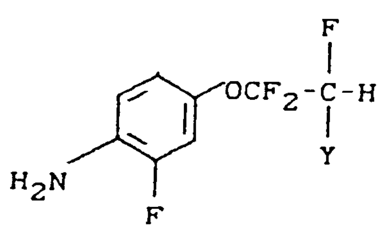

(I)

dans laquelle

Y représente du fluor, du chlore ou un groupe trifluorométhyle,

le composé 2-fluoro-4-tétrafluoréthoxyaniline étant exclu.

**2.** Procédé de production de composés de formule (I)

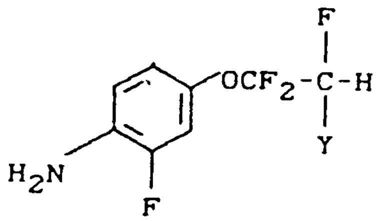

(I)

dans laquelle

Y représente le fluor, le chlore ou le groupe trifluorométhyle,

à l'exclusion du composé 2-fluoro-4-tétrafluoréthoxyaniline, caractérisé en ce qu'on fait réagir un fluorophénol de formule (II)

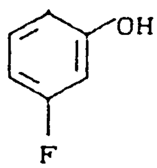

(II)

avec un composé trifluoréthylénique de formule (III)

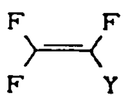

(III)

dans laquelle

Y a la définition indiquée pour la formule (I)

et on effectue la nitration du composé ainsi obtenu de formule (IV)

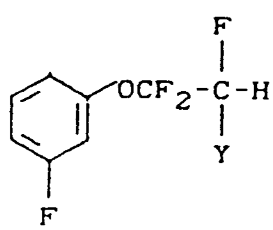

(IV)

dans laquelle

Y a la définition indiquée pour la formule 1, puis

on procède à une réduction.

**3.** Procédé de production de composés de formule (I), caractérisé en ce qu'on fait réagir un composé de formule (V)

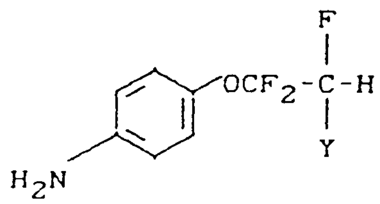

(V)

dans laquelle

Y a la définition indiquée pour la formule (I),

avec du nitrite du sodium en présence d'un acide protonique contenant du fluor, de manière à obtenir un composé de formule (IV) qu'on nitre et qu'on réduit ensuite.